(19) Europäisches Patentamt
European Patent Office
Office européen des brevets

(11) Numéro de publication : 0 323 325 B1

(12) # FASCICULE DE BREVET EUROPEEN

(45) Date de publication du fascicule du brevet : **16.10.91 Bulletin 91/42**

(21) Numéro de dépôt : **88403289.7**

(22) Date de dépôt : **22.12.88**

(51) Int. Cl.[5] : **C07D 491/052,** C07D 495/04, C07D 491/048, A61K 31/50, // (C07D491/052, 311:00, 237:00), (C07D495/04, 335:00, 237:00), (C07D491/04, 307:00, 237:00)

(54) **Dérivés tricycliques agonistes des récepteurs cholinergiques, procédé pour leur préparation et médicaments en contenant.**

(30) Priorité : **24.12.87 FR 8718187**

(43) Date de publication de la demande : **05.07.89 Bulletin 89/27**

(45) Mention de la délivrance du brevet : **16.10.91 Bulletin 91/42**

(84) Etats contractants désignés : **AT BE CH DE ES FR GB GR IT LI LU NL SE**

(56) Documents cités :
**EP-A- 0 251 937**
**FR-A- 2 141 697**

(73) Titulaire : **SANOFI**
**40, Avenue George V**
**F-75008 Paris (FR)**

(72) Inventeur : **Wermuth, Camille Georges**
**3, Rue de la Côte d'Azur**
**F-67100 Strasbourg (FR)**
Inventeur : **Worms, Paul**
**10, Rue du Devois**
**F-34980 St Gely Du Fesc (FR)**
Inventeur : **Bourguignon, Jean-Jacques**
**2, Rue Feldwasser**
**F-67150 Hipsheim (FR)**
Inventeur : **Brodin, Roger**
**26, Mas des Bruyères, Avenue de Monsieur Teste**
**F-34070 Montpellier (FR)**

(74) Mandataire : **Gillard, Marie-Louise et al**
**Cabinet Beau de Loménie 55, Rue d'Amsterdam**
**F-75008 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 323 325 B1

## Description

Les démences séniles et en particulier les démences de type Alzheimer sont des affections graves dont la fréquence tend à augmenter en fonction de l'augmentation de la longévité de la population.

Les études entreprises par différents auteurs, ont mis en évidence dans la maladie d'Alzheimer l'existence d'un déficit spécifique des marqueurs cholinergiques corticaux provoquant des troubles graves des fonctions supérieures.

Les résultats obtenus par utilisation d'agonistes muscariniques pour le traitement des démences séniles se sont montrés encourageants. Toutefois les agonistes muscariniques n'existent qu'en petit nombre et se sont avérés de maniement difficile chez l'homme.

Par suite la recherche d'agonistes muscariniques post-synaptiques comme traitement de la maladie d'Alzheimer s'avère aujourd'hui tout à fait souhaitable.

L'intérêt de disposer d'agonistes muscariniques centraux sélectifs pour remédier au déficit cholinergique dans la maladie d'Alzheimer a été mentionné notamment dans ISI Atlas of Science : Pharmacology (1987), p. 98 à 100.

C'est à ce problème que la présente invention tente d'apporter une solution par de nouveaux produits actifs sélectivement sur les récepteurs muscariniques centraux $M_1$.

Selon un premier aspect, la présente invention a pour objet des composés tricycliques nouveaux répondant à la formule générale :

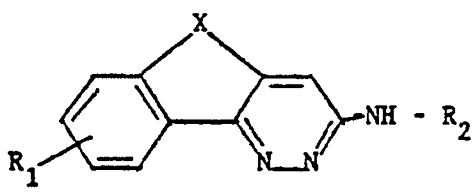

(I)

dans laquelle :

— X représente un atome d'oxygène ou de soufre ou encore X représente un groupe $-OCH_2-$ ou un groupe $-SCH_2-$,

— $R_1$ représente l'hydrogène ou un atome d'halogène de préférence le chlore,

— $R_2$ représente :

* un groupe

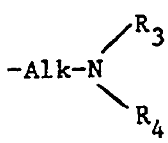

dans lequel :

- Alk représente un groupe alkylène droit ou ramifié ayant de 2 à 5 atomes de carbone,
- $R_3$ et $R_4$ représentent chacun indépendamment l'hydrogène, un groupe alkyle inférieur ayant 1 à 4 atomes de carbone ou encore $R_3$ et $R_4$ constituent avec l'atome d'azote auquel ils sont liés un groupe amino cyclique à 5 ou 6 chaînons comportant éventuellement un second hétéroatome et notamment les groupes pyrrolidinyle-1, pipéridino, morpholino, ou pipérazinyle-1 :

* un groupe

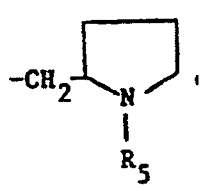

où $R_5$ représente un groupe alkyle inférieur ayant 1 à 4 atomes de carbone ; ainsi que leurs sels d'addition avec les acides minéraux ou organiques pharmaceutiquement acceptables.

Selon un second aspect, l'invention a pour objet un procédé de préparation des composés de formule (I) qui peut être représenté par le schéma suivant :

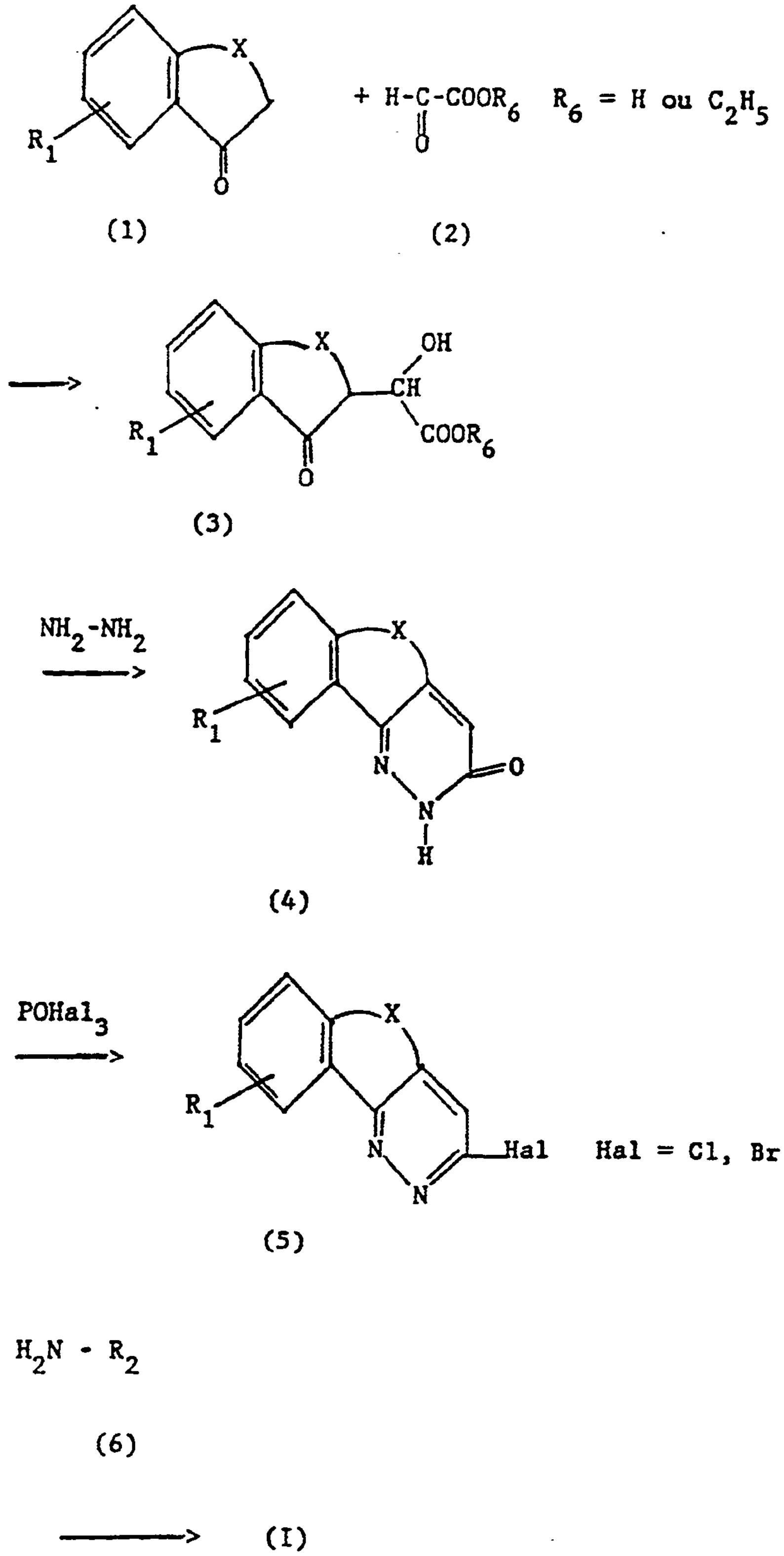

(1) (2) (3) (4) (5) (6) (I)

Par action du glyoxylate d'éthyle ou de l'acide glyoxylique (2) sur un cétohétérocycle (1) à température comprise entre 60 et 150°C, on forme l'hydroxyester ou l'hydroxyacide (3). Celui-ci contient le plus souvent un peu du produit de déshydratation correspondant (ester acrylique). On peut le purifier par chromatographie ou encore utiliser directement le produit brut pour l'étape suivante.

Le produit (3) par chauffage avec de l'hydrate d'hydrazine fournit la pyridazone (4). On opère soit avec un large excès d'hydrate d'hydrazine, soit au sein d'un solvant choisi parmi les solvants hydroxylés et notamment le n-butanol ou l'éthanol.

La pyridazone (4) traitée au reflux par un excès d'oxychlorure ou d'oxybromure de phosphore conduit au dérivé halogéné (5).

Enfin par chauffage du dérivé halogéné (5) avec le dérivé (6) au sein d'un solvant convenable on obtient le composé (I).

Le solvant peut être soit un solvant hydroxylé tel que le n-butanol, soit du diméthylformamide ou encore il peut être constitué par un excès du dérivé (6).

Lorsque la réaction de substitution du dérivé chloré s'avère lente, elle peut être facilitée par addition d'un activateur de la réaction, par exemple par addition de chlorure d'ammonium.

Eventuellement, les composés (I) ainsi obtenus peuvent être salifiés par un procédé connu.

Les produits de départ de formule (1) sont connus ou peuvent être préparés par des procédés connus.

Les exemples suivants sont fournis pour illustrer l'invention.

EXEMPLE 1.

Dichlorhydrate de (morpholino-2-éthylamino)-2 dihydro-9, 10 oxa-9 diaza-3,4 phénanthrène (SR 96094 A).

(I) X = $-O-CH_2-$ $R_1 = H$ $R_2 = -(CH_2)_2-$ 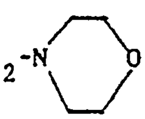

a) (oxo-4 chrommanyl-3) glycolate d'éthyle.

On chauffe pendant 9 heures à 135°C, le mélange de 14 g de chromannone-4 et 14,5 g de glyoxylate d'éthyle.

On chromatographie sur colonne de silice et en éluant avec le mélange hexane-acétate d'éthyle (80-20 vol/vol) on isole le produit attendu (3,6 g) sous forme de cristaux jaunes F : 50°C.

b) Oxo-2 tétrahydro-2,3,9,10 oxa-9 diaza-3,4 phénanthrène.

A la solution de 13 g d'ester préparé en a) dans 150 ml de n-butanol, on ajoute 2,78 ml d'hydrate d'hydrazine et agite le mélange pendant 3 heures à température ambiante puis chauffe au reflux pendant 24 heures.

Par refroidissement, il se forme des cristaux qu'on essore (3,45 g) F : > 250°C.

Au filtrat, on rajoute 1,4 ml d'hydrate d'hydrazine et chauffe au reflux pendant 24 heures.

Par refroidissement, on obtient 2,45 g du même produit.

c) Chloro-2 dihydro-9,10 oxa-9 diaza-3,4 phénanthrène.

On chauffe à 80°C pendant 1 heure 5 g du produit préparé ci-dessus et 75 ml d'oxychlorure de phosphore.

On verse goutte à goutte le mélange réactionnel sur un mélange glace-eau puis on alcalinise avec de la soude (solution à 33%).

On essore le précipité, lave abondamment avec de l'eau puis sèche sous vide.

On recristallise dans l'éthanol absolu.

On obtient 4,75 g du produit attendu F : 208°C.

d) SR 96094 A.

On chauffe à 120°C pendant 2 heures le mélange de 4,37 g du dérivé chloré préparé ci-dessus 13,1 ml de morpholino-2 éthylamine et 2,13 g de chlorure d'ammonium.

On verse le mélange réactionnel dans l'eau et on extrait avec de l'acétate d'éthyle. On extrait la phase organique avec une solution diluée d'acide chlorhydrique et sépare la phase aqueuse. On l'alcalinise avec du carbonate de potassium et extrait avec de l'acétate d'éthyle. On lave la solution avec de l'eau, sèche sur sulfate de sodium et évapore à siccité. On purifie par chromatographie sur colonne de silice. En éluant avec le mélange acétate d'éthyle-méthanol-ammoniaque (80-10-10 vol/vol) on obtient 2 g du produit attendu F : 149°C.

Dichlorhydrate

On dissout 1 g de la base obtenue ci-dessus dans l'isopropanol, et ajoute 0,55 ml de solution d'acide chlorhydrique concentré. On évapore à siccité et cristallise le résidu dans l'éthanol absolu. On obtient un solide jaune (0,9 g) F : 216°C.

Le dichlorhydrate cristallise avec 2 molécules d'eau.

EXEMPLE 2

Dichlorhydrate de (diéthylamino-2 éthylamino)-2 dihydro-9,10 thia-9 diaza-3,4 phénanthrène (SR 96056 A).

(I) X = $-S-CH_2-$ $R_1$ = H $R_2$ = 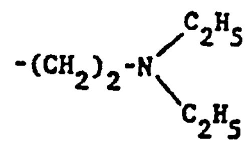

On opère comme dans l'exemple 1, en utilisant comme produit de départ la thiochromannone-4 au lieu de chromannone-4.

De la même façon, on obtient successivement :

a) l'(oxo-4 thiochromannyl-3) glycolate d'éthyle sous forme d'une huile Rendement 62%
b) l'oxo-2 tétrahydro-2,3,9,10 thia-9 diaza-3,4 phénanthrène F : > 250°C Rendement 32%
c) le chloro-2 dihydro-9,10 thia-9 diaza-3,4 phénanthrène F : 190°C Rendement 92%
d) SR 96056 A

Base : F : 146°C après recristallisation dans l'isopropanol
Dichlorhydrate : F : 162°C (isopropanol) Rendement 70%

Le dichlorhydrate cristallise avec 3 molécules d'eau.

EXEMPLE 3

Dichlorhydrate de (morpholino-2 éthylamino)-2 oxa-9 diaza-3,4 fluorène (SR 44288 A)

(I) X = O $R_1$ = H $R_2$ = 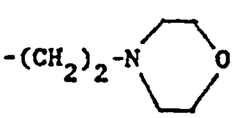

a) Oxo-2 dihydro-2,3 oxa-9 diaza-3,4 fluorène.

On chauffe pendant 4 heures à 80°C le mélange de 4 g d'oxo-3 dihydro-2,3 benzofuranne et 6,18 g d'acide glyoxylique.

On reprend le mélange réactionnel dans 60 ml d'éthanol absolu, ajoute 4,48 g d'hydrate d'hydrazine et chauffe au reflux pendant 65 heures. On évapore au bain-marie à siccité sous vide.

On dissout le résidu dans l'acétate d'éthyle puis filtre la solution sur colonne de silice en éluant avec le même solvant.

On chromatographie à 2 reprises sur une colonne de gel de silice en éluant la première fois avec le mélange chloroforme-méthanol 95-5 vol/vol puis avec le mélange chloroforme-méthanol 90-10 vol/vol. On obtient finalement 1,2 g du produit attendu F : > 260°C.

b) Chloro-2 oxa-9 diaza-3,4 fluorène.

On chauffe à 90°C, pendant 5 heures, le mélange de 1,2 g du produit préparé en a) et 30 ml d'oxychlorure de phosphore.

On évapore au bain-marie sous vide l'excès d'oxychlorure de phosphore et reprend le résidu dans l'eau glacée. On extrait avec de l'acétate d'éthyle, lave la solution organique avec de l'eau, sèche sur sulfate de

sodium et évapore le solvant sous vide.

On chromatographie le résidu sur colonne de gel de silice.

En éluant avec de l'acétate d'éthyle, on obtient le produit attendu (0,8 g) F : 122°C.

c) SR 44288 A

On chauffe au reflux pendant 140 heures, le mélange de 0.7 g du dérivé chloré obtenu ci-dessus de 1,34 g de morpholino-2 éthylamine dans 50 ml de n-butanol.

On traite comme indiqué dans l'exemple 1 d) et après chromatographie sur silice, (éluant chloroforme-méthanol 90-10), on obtient 0,4 g du produit attendu.

Dichlorhydrate

On dissout 0,4 g de base dans 5 ml d'éthanol absolu puis on ajoute 0,34 ml d'acide chlorhydrique concentré et laisse cristalliser.

On essore les cristaux, lave avec un peu d'éthanol et sèche sous vide. Poids 0,3 g F : > 260°C.

Le dichlorhydrate cristallise avec 1 molécule d'eau.

EXEMPLE 4 A 6

En opérant comme dans l'exemple 3 c) à partir du dérivé chloré obtenu dans l'exemple 3 b) mais en faisant varier l'amine utilisée, on obtient de la même façon les produits (I) réunis dans le tableau 1.

TABLEAU 1

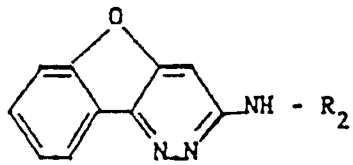

| Exemple n° | N° de code | $R_2$ | Sel isolé [Pt de fusion °C (solvant)] |
|---|---|---|---|
| 4 | SR 45040 A | 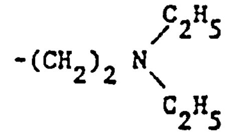 | Difumarate<br>F : 142-144 (éthanol) |
| 5 | SR 45041 A | 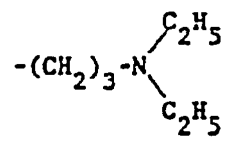 | Difumarate<br>F : 153-155 (éthanol) |
| 6 | SR 45042 A | 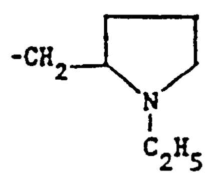 | Dichlorhydrate<br>(1 molécule d'eau)<br>F : 204-206 (éthanol) |

EXEMPLE 7

Dichlorhydrate de (morpholino-2 éthylamino)-2 thia-9 diaza-3,4 fluorène (SR 44289 A)

(I) X = S $R_1$ = H $R_2$ = 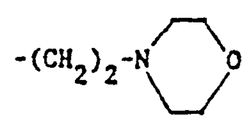

On opère comme dans l'exemple 3, en utilisant comme produit de départ l'oxo-3 dihydro-2,3 benzothiophène.

De la même façon, on obtient successivement :

a) l'oxo-2 dihydro-2,3 thia-9 diaza-3,4 fluorène F : > 260°C
b) le chloro-2 thia-9 diaza-3,4 fluorène F : 180°C
c) SR 44289 A

Base F : 138-140°C
Dichlorhydrate F : 236-238°C.

Le dichlorhydrate cristallise avec 0,5 molécule d'eau.

EXEMPLE 8

Fumarate de chloro-5 (diéthylamino-2 éthylamino)-2 oxa-9 diaza-3,4 fluorène

(I) X = O $R_1$ = 5-Cl $R_2$ = $(CH_2)_2$ 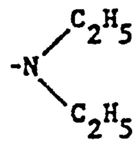

On opère comme dans l'exemple 3, en utilisant comme produit de départ le chloro-4 oxo-3 dihydro-2,3 benzofuranne.

De la même façon on prépare successivement :

a) le chloro-5 oxo-2 dihydro-2,3 oxa-9 diaza-3,4 fluorène F : > 260°C
b) le dichloro-2,5 oxa-9 diaza-3,4 fluorène F : 174-176°C
c) SR 45148 A isolé sous forme de fumarate F : 232-234°C (éthanol).

Les produits selon l'invention ont été étudiés en ce qui concerne leur action thérapeutique. Notamment l'intéraction des produits selon l'invention vis à vis des récepteurs muscariniques cholinergiques a été déterminée.

Chez les mammifères, il existe deux sous classes de récepteurs cholinergiques muscariniques : les récepteurs $M_1$ et $M_2$.

Les récepteurs de type $M_1$ sont concentrés dans certaines zones de cerveau telles que l'hippocampe, le cortex cérébral, le striatum ainsi que dans les ganglions sympathiques. Ces sites de liaison peuvent être sélectivement marqués par la [$^3$H] pirenzépine ([$^3$H]PZ). Ceux de type $M_2$ prédominent dans le coeur et dans l'iléon et peuvent être marqués par le [$^3$H] N-méthylscopolamine ([$^3$H]NMS). Afin de déterminer la sélectivité des produits de l'invention, vis-à-vis des sites $M_1$ et $M_2$, on a étudié leur intéraction in vitro avec les fixations à haute affinité de la [$^3$H] PZ et de la [$^3$H] NMS sur des membranes d'hippocampe de rat et de muscle lisse d'iléon de cobaye, respectivement.

METHODOLOGIES

A) Recherche d'une affinité pour le récepteur cholinergique muscarinique de type $M_1$.

L'intéraction des molécules avec les récepteurs muscariniques de type $M_1$ a été étudiée en mesurant in vitro sur un homogénat d'hippocampe de rat, le déplacement de la pirenzépine tritiée ([$^3$H]PZ) de ses sites de fixation spécifiques. Des aliquots (10 µl) d'un homogénat d'hippocampe de rat à 5% (P/v) dans un tampon $Na_2HPO_4$ (50 mM, pH 7,40), sont incubés 2 h à 4°C en présence de [$^3$H]PZ (76 Ci/nmole ; 1 nM final) et de concentrations croissantes en produit à étudier. Le volume final est de 2 ml. La réaction est arrêtée par centrifugation 10 min à 50.000 xg. Après décantation et lavage des culots, la radioactivité fixée est comptée par scintillation liquide. La fixation non spécifique est déterminée en présence de 10 µM de sulfate d'atropine. La concentration inhibitrice 50 ($CI_{50}$) est déterminée graphiquement. (Réf. : Watson J.D., Roeskoe W.R. and Yamamura H.I., Life Sci., 31, 2019-2029, 1982).

B) Recherche d'une affinité pour le récepteur cholinergique muscarinique de type $M_2$.

L'intéraction avec les récepteurs muscariniques de type $M_2$ a été étudiée en mesurant in vitro sur un homogénat de muscle lisse d'iléon de cobaye, le déplacement de la N-méthyl-scopolamine tritiée ([$^3$H]NMS) de ses sites de fixation spécifiques. Des aliquots (50 µl) d'un homogénat de muscle lisse d'iléon de cobaye à 0,625% (P/v) dans du tampon MEPES (20 mM) contenant NaCl (100 mM) et Mg $Cl_2$ (10 mM) (pH final : 7,5), sont incubés 20 min à 30°C en présence de [$^3$H]NMS (85 Ci/nmole ; 0,3 nM final) et de concentration croissantes en produits à tester. Le volume final est de 1 ml. La réaction est arrêtée par centrifugation 5 min à 15.000 xg. La fixation non spécifique est déterminée en présence de 10 µM de sulfate d'atropine.

(Réf. : Hammer R., Berrie C.P., Birdsall N.I.M., Burgen A.S.V. and Hulme E.C., Nature, 283, 90-92, 1980
Hulme E.C., Birdsall, N.I.M., Burgen A.S.V. and Mettha P., Mol. Pharmacol., 14, 737-750, 1978).

RESULTATS

Le tableau 2 indique les affinités des produits de l'invention pour les récepteurs $M_1$ et $M_2$. Les résultats sont exprimés en concentrations inhibitrices 50 pour cent (CI 50), soit la concentration (en µM) qui induit un déplacement de 50% du ligand tritié fixé aux récepteurs membranaires. La $CI_{50}$ de déplacement de la $^3$H-pirenzépine représente l'affinité pour le récepteur $M_1$ ; la CI50 de déplacement de la $^3$H-NMS représente l'affinité pour le récepteur $M_2$.

De plus dans la 3e colonne on a indiqué le rapport r entre les $CI_{50}$ $M_1$ et $M_2$ qui exprime la sélectivité des produits vis à vis de l'un des types de récepteurs.

TABLEAU 2

| Produit N° | $^3$H-Pirenzepine ($M_1$) CI 50, µ M | $^3$H-NMS ($M_2$) CI 50, µ M | r = ($M_2$/$M_1$) |
|---|---|---|---|
| SR 44288 A | 3 | >100 | >33 |
| SR 44289 A | 3,5 | 100 | 28 |
| SR 45040 A | 0,7 | 45 | 64 |
| SR 45041 A | 0,1 | 10 | 100 |
| SR 45042 A | 0,1 | 50 | 500 |
| SR 96056 A | 0,15 | 3,6 | 24 |
| SR 96094 A | 3,6 | >100 | >27 |

Ces résultats montrent que les composés selon l'invention présentent une forte affinité pour les récepteurs cholinergiques muscariniques avec une spécifité marquée pour les récepteurs centraux de type $M_1$.

Par ailleurs les composés selon l'invention ont été soumis à une étude pharmacologique in vivo.

ETUDE PHARMACOLOGIQUE IN VIVO

La pirenzépine (PZ) est un antagoniste spécifique des récepteurs chlolinergiques muscariniques centraux $M_1$. L'injection intrastriatale de PZ chez la souris induit un comportement rotatoire. On a étudié l'antagonisme de ce comportement par les produits selon l'invention.

Les produits selon l'invention sont injectés par voie intrapéritonéale (i.p.) après avoir été solubilisés dans l'eau distillée ou mis en suspension dans une solution de gomme arabique à 5%. Les contrôles sont réalisés par injection du solvant pur dans les mêmes conditions.

Les animaux utilisés sont des souris femelles (Swiss, CD 1, Charles River, France) de poids corporel compris entre 25 et 30 grammes.

La pirenzépine est mise en solution dans un tampon phosphate, le pH de la solution est 6.

Les produits à étudier ou leur solvants sont injectés par voie i.p. sous un volume de 0,4 ml pour 20 g de poids corporel, 15 minutes avant une injection directe de pirenzépine à la dose de 1µl de solvant dans le stratium droit de la souris, selon la méthode décrite par P. WORMS et al. dans Eur. J. Pharmacol., 1986, 121, 395-401.

Le nombre de rotations contralatérales (sens opposé au côté de l'injection) a été compté pendant trois périodes de 2 minutes après injection de pirenzépine : minutes 2 à 4, 8 à 10 et 13 à 15. Chaque traitement comporte 1 à 3 doses et 10 animaux par dose. Pour chaque traitement, on calcule le nombre total de rotations et le pourcentage d'antagonisme vis-à-vis du lot contrôle.

Les résultats sont reportés dans le tableau 3.

TABLEAU 3

| Produits n° | Antagonisme Pirenzépine à 3 mg/kg, I.P | 10 mg/kg, I.P | 30 mg/kg I.P |
|---|---|---|---|
| SR 44288 A | -32* | -52** | -100** |
| SR 44289 A | -42* | -85** | - |
| SR 45040 A | -47** | - | - |
| SR 45041 A | -72** | - | - |
| SR 45042 A | -52** | - | - |
| SR 96056 A | -14 | -55** | -79** |
| SR 96094 A | - 8 | -20* | -85** |

* $p < 0,05$ ** $p < 0,01$ vs témoins.

Enfin les composés selon l'invention n'ont manifesté aucun signe de toxicité apparente aux doses où ils sont actifs.

Par suite les composés (I) peuvent être utilisés en tant que médicaments et notamment dans les cas où se manifeste un déficit cholinergique cortical et en particulier dans le cas des démences de type Alzheimer.

En conséquence selon un autre de ses aspects la présente demande concerne les compositions pharmaceutiques contenant au moins un des composés de formule (I) ou un de leurs sels en tant qu'ingrédient actif.

Dans les compositions pharmaceutiques de la présente invention pour l'administration orale, sublinguale, sous-cutanée, intramusculaire, intraveineuse, transdermique ou rectale, les ingrédients actifs de formule I ci-dessus peuvent être administrés sous forme unitaires d'administration, en mélange avec des supports pharmaceutiques classiques, aux êtres humains notamment pour le traitement des démences séniles. Les formes unitaires d'administration appropriées comprennent les formes par voie orale,telles que les comprimés, les gélules, les poudres, les granulés et les solutions ou suspensions orales, les formes d'administration sublingale et buccale, les formes d'administration sous-cutanée, intramusculaire ou intraveineuse et les formes d'administration rectale.

Afin d'obtenir l'effet désiré, la dose de principe actif peut varier entre 50 et 2000 mg par jour.

Chaque dose unitaire peut contenir de 10 à 500 mg d'ingrédient actif en combinaison avec un support pharmaceutique. Cette dose unitaire peut être administrée 1 à 4 fois par jour.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Les poudres ou les granulés dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou

plusieurs supports ou additifs.

Ainsi à titre d'exemple, on peut préparer des gélules à base d'un des composés des exemples 1 à 8 ayant la composition suivante :

| | |
|---|---|
| Principe actif | 25 mg |
| Lactose | 110 mg |
| Stéarate de magnésium | 5 mg |

en mélangeant intimement les ingrédients ci-dessus et en versant le mélange dans des gélules de gélatine dure.

De telles compositions pharmaceutiques constituent un objet de l'invention.

Leur procédé de préparation en constitue un autre.

Il consiste à mélanger à un véhicule pharmaceutiquement acceptable une dose efficace d'au moins un composé de formule (I).

## Revendications

### Revendications pour les Etats contractants suivants : AT, BE, CH, DE, FR, GB, IT, LI, LU, NL, SE

1. Nouveaux composés tricycliques de formule générale :

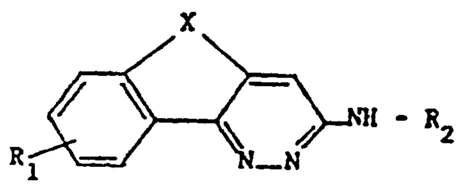

(I)

dans laquelle :

— X représente un atome d'oxygène ou de soufre ou encore X représente un groupe —$OCH_2$— ou un groupe —$SCH_2$—,

— $R_1$ représente l'hydrogène ou un atome d'halogène de préférence le chlore,

— $R_2$ représente :

* un groupe

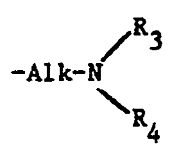

dans lequel :

• Alk représente un groupe alkylène droit ou ramifié ayant de 2 à 5 atomes de carbone,

• $R_3$ et $R_4$ représentent chacun indépendamment l'hydrogène, un groupe alkyle inférieur ayant 1 à 4 atomes de carbone ou encore $R_3$ et $R_4$ constituent avec l'atome d'azote auquel ils sont liés un groupe amino cyclique à 5 ou 6 chaînons comportant éventuellement un second hétéroatome, ou

* un groupe

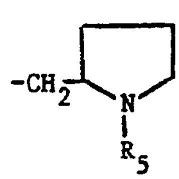

où $R_5$ représente un groupe alkyle inférieur ayant 1 à 4 atomes de carbone ; ainsi que leurs sels avec

les acides minéraux ou organiques pharmaceutiquement acceptables.

2. Composés selon la revendication 1, de formule (I) dans laquelle le groupe $R_2$ désigne

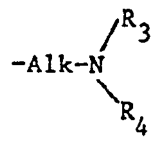 dans lequel 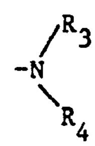

représente un groupe pyrrolidinyle-1, pipéridino, morpholino, pipérazinyle-1.

3. Procédé de préparation des composés de formule (I) tels que définis à la revendication 1, caractérisé en ce que :

— on traite à chaud entre 60 et 150°C un cétohétérocycle par le glyoxylate d'éthyle ou l'acide glyoxylique pour former l'hydroxyester ou l'hydroxyacide :

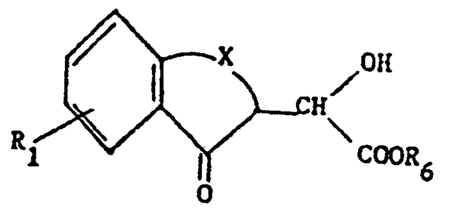

où $R_6$ est H ou $C_2H_5$

(a)

— on chauffe cet hydroxyester ou hydroxyacide avec de l'hydrate d'hydrazine, soit avec un large excès dudit hydrate d'hydrazine, soit au sein d'un solvant convenable pour former la pyridazone

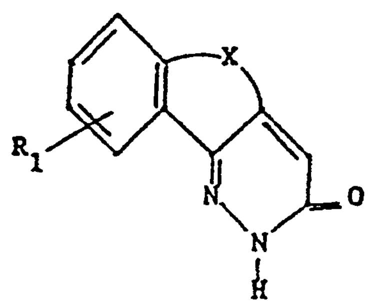

(b)

— on traite au reflux la pyridazone par un excès d'oxychlorure ou d'oxybromure de phosphore pour former le dérivé halogéné correspondant

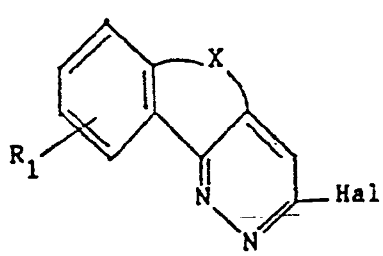

Hal = Cl, Br

(c)

— on substitue ledit dérivé halogéné par chauffage avec un dérivé de formule $H_2N\text{-}R_2$, soit en présence d'un excès dudit dérivé de formule $H_2N\text{-}R_2$, soit au sein d'un solvant convenable, éventuellement en présence d'un activateur de la réaction, pour former le composé (I) correspondant ;

— éventuellement on salifie le composé (I) selon un procédé connu.

4. Procédé selon la revendication 3, caractérisé en ce que l'hydrate d'hydrazine est chauffé avec l'hydroxyester ou l'hydroxyacide (a) au sein d'un solvant hydroxylé, tel que le n-butanol ou l'éthanol.

5. Procédé selon l'une des revendications 3 ou 4, caractérisé en ce que la réaction du dérivé halogéné (c) avec le dérivé $H_2N\text{-}R_2$ s'effectue au sein d'un solvant hydroxylé tel que le n-butanol ou au sein du diméthylformamide.

6. Procédé selon l'une quelconque des revendications 3 à 5, caractérisé en ce que la réaction du dérivé halogéné (c) avec le dérivé $H_2N-R_2$ s'effectue en présence de chlorure d'ammonium.

7. Compositions pharmaceutiques contenant à titre de principe actif, au moins un composé de formule (I), tel que défini à la revendication 1, en combinaison avec un véhicule pharmaceutiquement acceptable.

**Revendications pour l'Etat contractant suivant : GR**

1. Nouveaux composés tricycliques de formule générale :

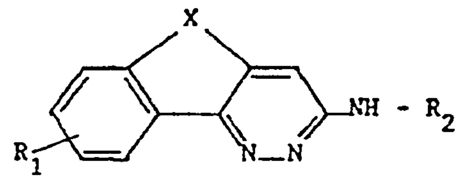

(I)

dans laquelle :

— X représente un atome d'oxygène ou de soufre ou encore X représente un groupe $-OCH_2-$ ou un groupe $-SCH_2-$,

— $R_1$ représente l'hydrogène ou un atome d'halogène de préférence le chlore,

— $R_2$ représente :

* un groupe

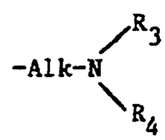

dans lequel :

• Alk représente un groupe alkylène droit ou ramifié ayant de 2 à 5 atomes de carbone,

• $R_3$ et $R_4$ représentent chacun indépendamment l'hydrogène, un groupe alkyle inférieur ayant 1 à 4 atomes de carbone ou encore $R_3$ et $R_4$ constituent avec l'atome d'azote auquel ils sont liés un groupe amino cyclique à 5 ou 6 chaînons comportant éventuellement un second hétéroatome, ou

* un groupe

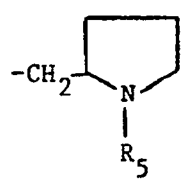

où $R_5$ représente un groupe alkyle inférieur ayant 1 à 4 atomes de carbone ; ainsi que leurs sels avec les acides minéraux ou organiques pharmaceutiquement acceptables.

2. Composés selon la revendication 1, de formule (I) dans laquelle le groupe $R_2$ désigne

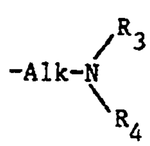 dans lequel 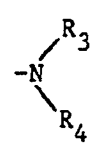

représente un groupe pyrrolidinyle-1, pipéridino, morpholino, pipérazinyle-1.

3. Procédé de préparation des composés de formule (I) tels que définis à la revendication 1, caractérisé en ce que :

— on traite à chaud entre 60 et 150°C un cétohétérocycle par le glyoxylate d'éthyle ou l'acide glyoxylique

pour former l'hydroxyester ou l'hydroxyacide :

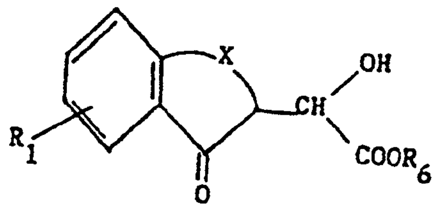

où $R_6$ est H ou $C_2H_5$

(a)

— on chauffe cet hydroxyester ou hydroxyacide avec de l'hydrate d'hydrazine, soit avec un large excès dudit hydrate d'hydrazine, soit au sein d'un solvant convenable pour former la pyridazone

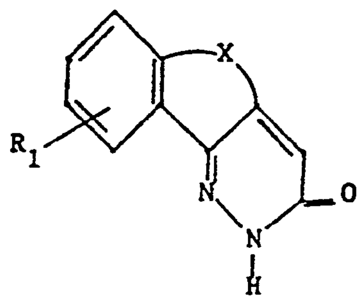

(b)

— on traite au reflux la pyridazone par un excès d'oxychlorure ou d'oxybromure de phosphore pour former le dérivé halogéné correspondant

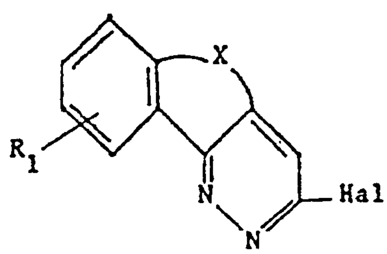

Hal = Cl, Br

(c)

— on substitue ledit dérivé halogéné par chauffage avec un dérivé de formule $H_2N-R_2$, soit en présence d'un excès dudit dérivé de formule $H_2N-R_2$, soit au sein d'un solvant convenable, éventuellement en présence d'un activateur de la réaction, pour former le composé (I) correspondant ;

— éventuellement on salifie le composé (I) selon un procédé connu.

4. Procédé selon la revendication 3, caractérisé en ce que l'hydrate d'hydrazine est chauffé avec l'hydroxyester ou l'hydroxyacide (a) au sein d'un solvant hydroxylé, tel que le n-butanol ou l'éthanol.

5. Procédé selon l'une des revendications 3 ou 4, caractérisé en ce que la réaction du dérivé halogéné (c) avec le dérivé $H_2N-R_2$ s'effectue au sein d'un solvant hydroxylé tel que le n-butanol ou au sein du diméthylformamide.

6. Procédé selon l'une quelconque des revendications 3 à 5, caractérisé en ce que la réaction du dérivé halogéné (c) avec le dérivé $H_2N-R_2$ s'effectue en présence de chlorure d'ammonium.

7. Procédé pour la préparation de compositions pharmaceutiques, caractérisé en ce qu'il consiste à mélanger à un véhicule pharmaceutiquement acceptable une dose efficace d'au moins un composé de formule (I), tel que défini à la revendication 1.

**Revendications pour l'Etat contractant suivant : ES**

1. Procédé pour la préparation de nouveaux composés tricycliques de formule générale :

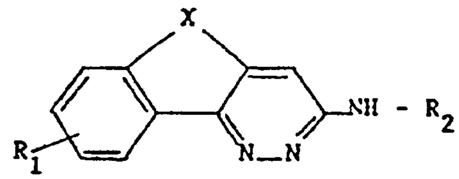 (I)

dans laquelle :

— X représente un atome d'oxygène ou de soufre ou encore X représente un groupe —$OCH_2$— ou un groupe —$SCH_2$—,

— $R_1$ représente l'hydrogène ou un atome d'halogène de préférence le chlore,

— $R_2$ représente :

* un groupe

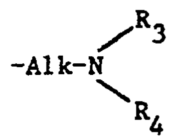

dans lequel :

• Alk représente un groupe alkylène droit ou ramifié ayant de 2 à 5 atomes de carbone,

• $R_3$ et $R_4$ représentent chacun indépendamment l'hydrogène, un groupe alkyle inférieur ayant 1 à 4 atomes de carbone ou encore $R_3$ et $R_4$ constituent avec l'atome d'azote auquel ils sont liés un groupe amino cyclique à 5 ou 6 chaînons comportant éventuellement un second hétéroatome, ou

* un groupe

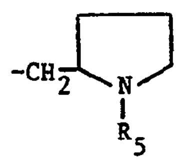

où $R_5$ représente un groupe alkyle inférieur ayant 1 à 4 atomes de carbone ; ainsi que de leurs sels avec les acides minéraux ou organiques pharmaceutiquement acceptables, caractérisé en ce qu'il consiste à :

— traiter à chaud entre 60 et 150°C un cétohétérocycle par le glyoxylate d'éthyle ou l'acide glyoxylique pour former l'hydroxyester ou l'hydroxyacide :

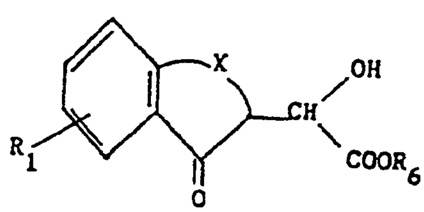 où $R_6$ est H ou $C_2H_5$

(a)

— chauffer cet hydroxyester ou hydroxyacide avec de l'hydrate d'hydrazine, soit avec un large excès dudit hydrate d'hydrazine, soit au sein d'un solvant convenable pour former la pyridazone

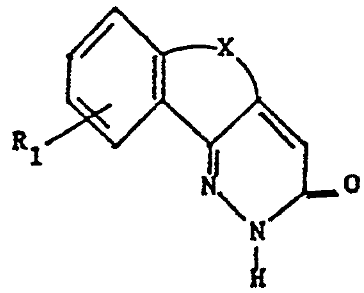

(b)

— traiter au reflux la pyridazone par un excès d'oxychlorure ou d'oxybromure de phosphore pour former le dérivé halogéné correspondant

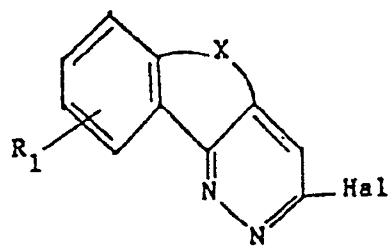

Hal = Cl, Br

(c)

— substituer ledit dérivé halogéné par chauffage avec un dérivé de formule $H_2N-R_2$, soit en présence d'un excès dudit dérivé de formule $H_2N-R_2$, soit au sein d'un solvant convenable, éventuellement en présence d'un activateur de la réaction, pour former le composé (I) correspondant ;

— éventuellement salifier le composé (I) selon un procédé connu.

2. Procédé selon la revendication 1, caractérisé en ce que ledit dérivé halogéné (c) est substitué par chauffage avec un dérivé de formule $H_2N-R_2$ dans laquelle $R_2$ représente le groupe :

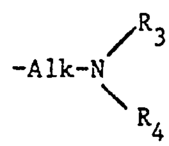 dans lequel 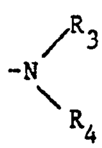

représente un groupe pyrrolidinyle-1, pipéridino, morpholino, pipérazinyle-1.

3. Procédé selon l'une des revendications 1 ou 2, caractérisé en ce que l'hydrate d'hydrazine est chauffé avec l'hydroxyester ou l'hydroxy-acide (a) au sein d'un solvant hydroxylé, tel que le n-butanol ou l'éthanol.

4. Procédé selon l'une des revendications 1 à 3, caractérisé en ce que la réaction du dérivé halogéné (c) avec le dérivé $H_2N-R_2$ s'effectue au sein d'un solvant hydroxylé tel que le n-butanol ou au sein du diméthylformamide.

5. Procédé selon l'une quelconque des revendications 1 à 4, caractérisé en ce que la réaction du dérivé halogéné (c) avec le dérivé $H_2N-R_2$ s'effectue en présence de chlorure d'ammonium.

6. Procédé pour la préparation de compositions pharmaceutiques, caractérisé en ce qu'il consiste à mélanger à un véhicule pharmaceutiquement acceptable, une dose efficace d'au moins un composé de formule (I) préparé selon l'une quelconque des revendications 1 à 5.

## Claims

### Claims for the following Contracting States : AT, BE, CH, DE, FR, GB, IT, LI, LU, NL, SE

1. Novel tricyclic compounds of the general formula

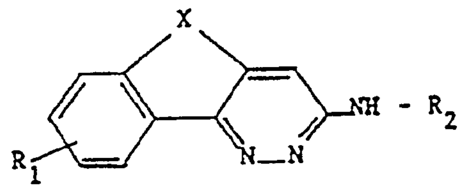

(I)

in which

— X represents an oxygen or sulfur atom or X represents a group —$OCH_2$— or a group —$SCH_2$— ;

— $R_1$ represents hydrogen or a halogen atom, preferably chlorine ; and

— $R_2$ represents

* a group

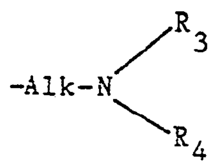

,

in which

• Alk represents a linear or branched alkylene group having from 2 to 5 carbon atoms, and

• $R_3$ and $R_4$ each independently represent hydrogen or a lower alkyl group having 1 to 4 carbon atoms ; or $R_3$ and $R_4$, with the nitrogen atom to which they are bonded, form a 5- or 6-membered cyclic amino group optionally containing a second heteroatom ; or

* a group

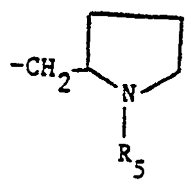

in which $R_5$ represents a lower alkyl group having 1 to 4 carbon atoms, and their salts with pharmaceutically acceptable mineral or organic acids.

2. Compounds according to claim 1, of formula (I) in which the group $R_2$ denotes

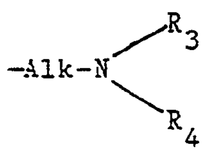

, in which

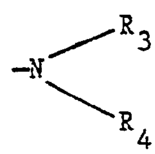

represents a pyrrolidin-1-yl, piperidino, morpholino or piperazin-1-yl group.

3. Process for the preparation of the compounds of formula (I), such as defined in claim 1, characterized in that :

— a ketoheterocycle is heat-treated, at between 60 and 150°C, with ethyl glyoxylate or glyoxylic acid to form the hydroxyester or hydroxyacid :

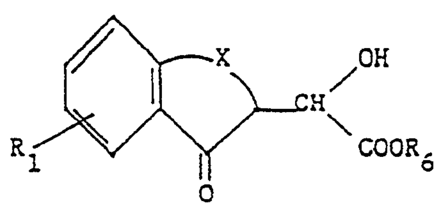

(a)

in which $R_6$ is H or $C_2H_5$,

— this hydroxyester or hydroxyacid is heated with hydrazine hydrate either with a large excess of hydrazine hydrate or in a suitable solvent to form the pyridazone :

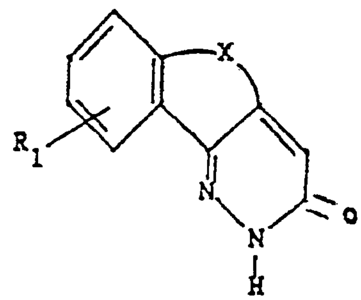

(b)

— the pyridazone is treated under reflux with an excess of phosphorus oxychloride or oxybromide to form the corresponding halogen derivative :

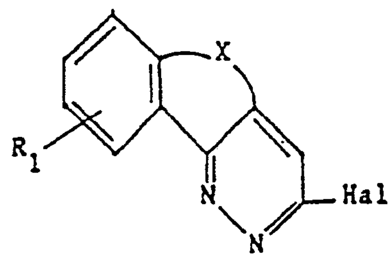

(c)

in which Hal = Cl or Br,

— Said halogen derivative is substituted by treating with a derivative $H_2N\text{-}R_2$, either with an excess of said derivative $H_2N\text{-}R_2$ or in a suitable solvent, if appropriate in the presence of a reaction activator, to form the corresponding compounds (I), and

— if desired, the compound (I) is converted to a salt by a known process.

4. Process according to claim 3, characterized in that the hydrazine hydrate is heated with the hydroxyester or the hydroxyacid (a) within a hydroxylated solvent, such as n-butanol or ethanol.

5. Process according to one of claims 3 or 4, characterized in that the reaction of the halogenated derivative (c) with the $H_2N\text{-}R_2$ derivative occurs within a hydroxylated solvent such as n-butanol or within dimethylformamide.

6. Process according to any one of claims 3 to 5, characterized in that the reaction of the halogenated derivative (c) with the $H_2N\text{-}R_2$ derivative occurs in the presence of ammonium chloride.

7. Pharmaceutical compositions in which at least a compound of formula (I) is present as the active principle, in combination with a pharmaceutically acceptable vehicle.

**Claims for the following Contracting State : GR**

1. Novel tricyclic compounds of the general formula

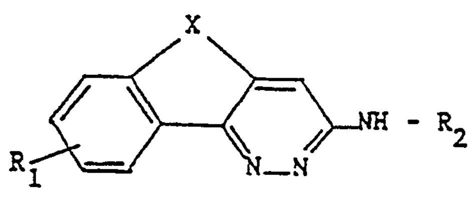

(I)

in which

— X represents an oxygen or sulfur atom or X represents a group —$OCH_2$— or a group —$SCH_2$— ;

— $R_1$ represents hydrogen or a halogen atom, preferably chlorine ; and

— $R_2$ represents

* a group

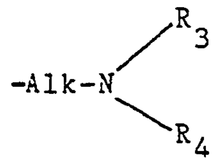,

in which

- Alk represents a linear or branched alkylene group having from 2 to 5 carbon atoms, and
- $R_3$ and $R_4$ each independently represent hydrogen or a lower alkyl group having 1 to 4 carbon atoms ; or $R_3$ and $R_4$, with the nitrogen atom to which they are bonded, form a 5- or 6-membered cyclic amino group optionally containing a second heteroatom ; or

* a group

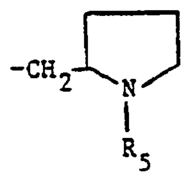

in which $R_5$ represents a lower alkyl group having 1 to 4 carbon atoms, and their salts with pharmaceutically acceptable mineral or organic acids.

2. Compounds according to claim 1, of formula (I) in which the group $R_2$ denotes

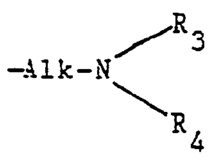, in which 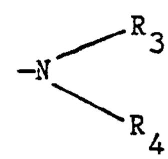

represents a pyrrolidin-1-yl, piperidino, morpholino or piperazin-1-yl group.

3. Process for the preparation of the compounds of formula (I), such as defined in claim 1, characterized in that :

— a ketoheterocycle is heat treated, at between 60 and 150°C, with ethyl glyoxylate or glyoxylic acid to form the hydroxyester or hydroxyacid :

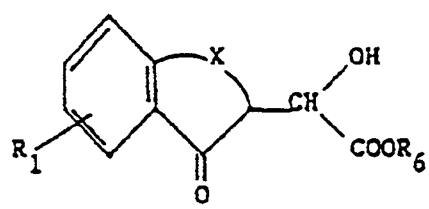 (a)

in which $R_6$ is H or $C_2H_5$,

— this hydroxyester or hydroxyacid is heated with hydrazine hydrate either with a large excess of hydrazine hydrate or in a suitable solvent to form the pyridazone :

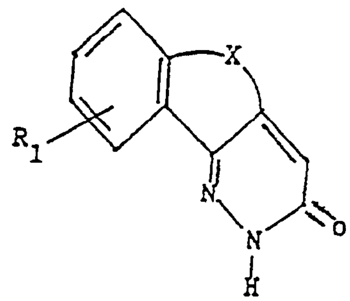

(b),

— the pyridazone is treated under reflux with an excess of phosphorus oxychloride or oxybromide to form the corresponding halogen derivative :

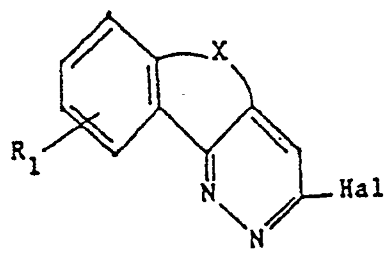

(c)

in which Hal = Cl or Br,

— Said halogen derivative is substituted by heating with a derivative $H_2N$-$R_2$, either with an excess of said derivative $H_2N$-$R_2$ or in a suitable solvent, if appropriate in the presence of a reaction activator, to form the corresponding compounds (I), and

— if desired, the compound (I) is converted to a salt by a known process.

4. Process according to claim 3, characterized in that the hydrazine hydrate is heated with the hydroxyester or the hydroxyacid (a) within a hydroxylated solvent, such as n-butanol or ethanol.

5. Process according to one of claims 3 or 4, characterized in that the reaction of the halogenated derivative (c) with the $H_2N$-$R_2$ derivative occurs within a hydroxylated solvent such as n-butanol or within dimethylformamide.

6. Process according to any one of claims 3 to 5, characterized in that the reaction of the halogenated derivative (c) with the $H_2N$-$R_2$ derivative occurs in the presence of ammonium chloride.

7. Process for the preparation of pharmaceutical compositions, characterized in that it consists in mixing with a pharmaceutically acceptable vehicle an efficient dose of at least a compound of formula (I), such as defined in claim 1.

**Claims for the following Contracting State : ES**

1. Process or the preparation of novel tricyclic compounds of general formula :

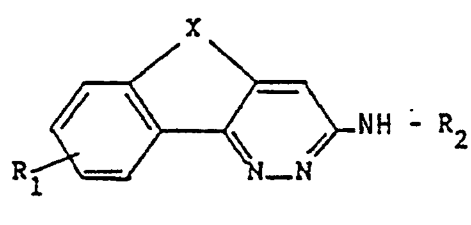

(I)

in which :

— X represents an oxygen or sulfur atom or X represents a group —$OCH_2$— or a group —$SCH_2$— ;

— $R_1$ represents hydrogen or a halogen atom, preferably chlorine ; and

— $R_2$ represents :

* a group

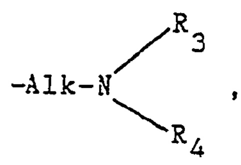

in which :

- Alk represents a linear or branched alkylene group having from 2 to 5 carbon atoms, and
- $R_3$ and $R_4$ each independently represent hydrogen or a lower alkyl group having 1 to 4 carbon atoms ; or $R_3$ and $R_4$, with the nitrogen atom to which they are bonded, form a 5- or 6-membered cyclic amino group optionally containing a second heteroatom ; or

* a group

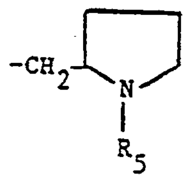

in which $R_5$ represents a lower alkyl group having 1 to 4 carbon atoms, and their salts with pharmaceutically acceptable mineral or organic acids, characterized in that it consists in :

— heat-treating at between 60 and 150°C a ketoheterocycle with ethyl glyoxylate or glyoxylic acid to form the hydroxyester or hydroxyacid :

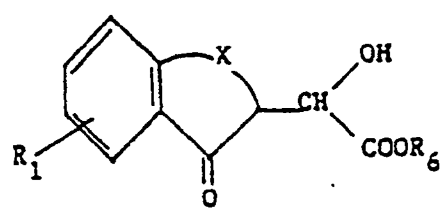

(a)

in which $R_6$ is H or $C_2H_5$ ;

— heating said hydroxyester or hydroxyacid with hydrazine hydrate either with a large excess of said hydrazine hydrate, or in a suitable solvent to form the pyridazone :

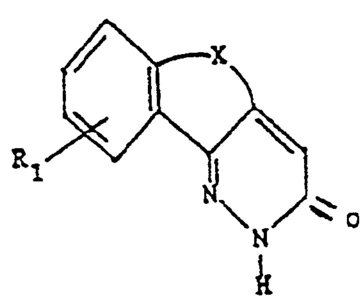

(b)

— treating the pyridazone under reflux with an excess of phosphorus oxychloride or oxybromide to form the corresponding halogen derivative :

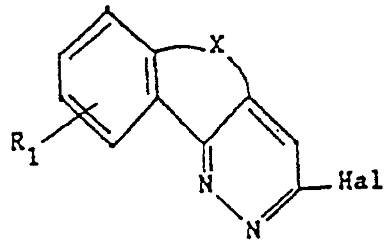

(c)

in which Hal = Cl or $B_1$ ;
— substituting said halogen derivative by heating with a derivative of formula $H_2N$-$R_2$, either with an excess of said derivative of formula $H_2N$-$R_2$, or in a suitable solvent, if appropriate in the presence of a reaction activator to form the corresponding compounds (I), and
— if desired, converting the compound (I) according to a known process.

2. Process according to claim 1, characterized in that said halogen derivative (c) is substituted by heating with a derivative of formula $H_2N$-$R_2$ in which $R_2$ represents the group :

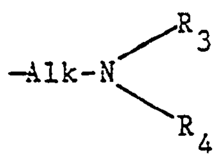

, in which

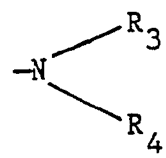

represents a pyrrolidin-1-yl, piperidino, morpholino or piperazin-1-yl group.

3. Process according to one of claims 1 or 2, characterized in that the hydrazine hydrate is heated with the hydroxyester on the hydroxyacid (a) within a hydroxylated solvent, such as n-butanol or ethanol.

4. Process according to one of claims 1 to 3, characterized in that the reaction of the halogenated derivative (c) with the $H_2N$-$R_2$ derivative occurs within a hydroxylated solvent such as n-butanol or within dimethylformamide.

5. Process according to any one of claims 1 to 4, characterized in that the reaction of the halogenated derivative (c) with the $H_2N$-$R_2$ derivative occurs in the presence of ammonium chloride.

6. Process for the preparation of pharmaceutical compositions, characterized in that it consists in mixing with a pharmaceutically acceptable vehicle an efficient dose of at least a compound of formula (I), prepared according to any one of claims 1 to 5.

**Patentansprüche**

**Patentansprüche für folgende Vertragsstaaten : AT, BE, CH, DE, FR, GB, IT, LI, LU, NL, SE**

1. Neue tricyclische Verbindungen der allgemeinen Formel

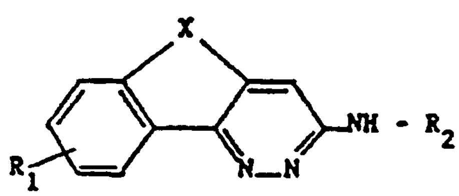

(I)

in welcher
— X ein Sauerstoff- oder Schwefelatom darstellt, oder aber X für eine Gruppe —$OCH_2$— oder eine Gruppe —$SCH_2$— steht,
— $R_1$ Wasserstoff oder ein Halogenatom, vorzugsweise Chlor, darstellt,
— $R_2$ darstellt :
* eine Gruppe

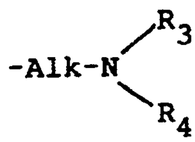,

worin :

- Alk für eine gerade oder verzweigte Alkylengruppe mit 2 bis 5 Kohlenstoffatomen steht,
- $R_3$ und $R_4$ jeweils unabhängig Wasserstoff, eine Niedrigalkylgruppe mit 1 bis 4 Kohlenstoffatomen darstellen, oder aber $R_3$ und $R_4$ mit dem Stickstoffatom, an das sie gebunden sind, eine cyclische Aminogruppe mit 5 oder 6 Ketten, die gegebenenfalls ein zweites Heteroatom umfaßt, bilden, oder

* eine Gruppe

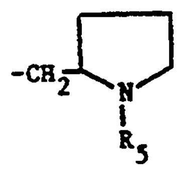,

worin $R_5$ eine Niedrigalkylgruppe mit 1 bis 4 Kohlenstoffatomen darstellt ; sowie deren Salze mit pharmazeutisch akzeptablen Mineral- oder organischen Säuren.

2. Verbindungen nach Anspruch 1 der Formel (I), in welcher die Gruppe $R_2$

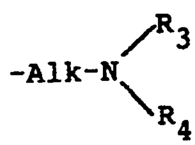 bezeichnet, worin 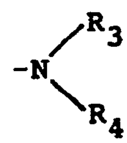

eine 1-Pyrrolidinyl-, Piperidino-, Morpholino- oder 1-Piperazinylgruppe darstellt.

3. Verfahren zur Herstellung der Verbindungen der Formel (I) nach Anspruch 1, dadurch gekennzeichnet, daß

— ein Ketoheterocyclus zwischen 60 und 150°C mit Ethylglyoxylat oder Glyoxylsäure hitzebehandelt wird zur Bildung des Hydroxyesters oder der Hydroxysäure

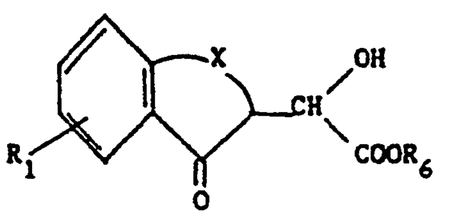, worin $R_6$ H oder $C_2H_5$ ist,
(a)

— dieser Hydroxyester oder diese Hydroxysäure mit Hydrazinhydrat erhitzt wird, u.zw. entweder mit einem großen Überschuß des Hydrazinhydrats oder in einem Lösungsmittel, das für die Bildung des Pyridazons

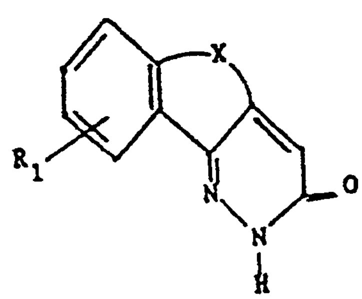

(b)

geeignet ist,

— das Pyridazon unter Rückfluß mit einem Überschuß an Phosphoroxychlorid oder -oxybromid behandelt wird, um das entsprechende Halogenderivat

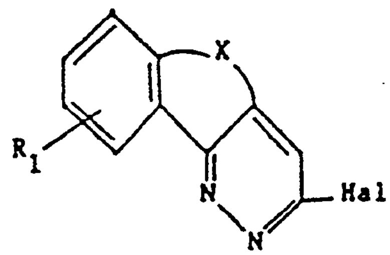

Hal = Cl, Br

(c)

zu bilden,

— das Halogenderivat durch Erhitzen mit einem Derivat der Formel $H_2N\text{-}R_2$ entweder in Gegenwart eines Überschusses des Derivats der Formel $H_2N\text{-}R_2$ oder in einem geeigneten Lösungsmittel, gegebenenfalls in Gegenwart eines Reaktionsaktivators zur Bildung der korrespondierenden Verbindung (I) substituiert wird ;

— gegebenenfalls die Verbindung (I) nach einem bekannten Verfahren in das Salz übergeführt wird.

4. Verfahren nach Anspruch 3, dadurch gekennzeichnet, daß das Hydrazinhydrat mit einem Hydroxyester oder einer Hydroxysäure (a) in einem Hydroxyllösungsmittel, wie n-Butanol oder Ethanol, erhitzt wird.

5. Verfahren nach einem der Ansprüche 3 oder 4, dadurch gekennzeichnet, daß die Reaktion des Halogenderivats (c) mit dem Derivat $H_2N\text{-}R_2$ in einem Hydroxyllösungsmittel, wie n-Butanol, oder in Dimethylformamid erfolgt.

6. Verfahren nach einem der Ansprüche 3 bis 5, dadurch gekennzeichnet, daß die Reaktion des Halogenderivats (c) mit dem Derivat $H_2N\text{-}R_2$ in Gegenwart von Ammoniumchlorid erfolgt.

7. Pharmazeutische Zusammensetzungen, die als Wirkstoff mindestens eine Verbindung der Formel (I) gemäß Anspruch 1 in Kombination mit einem pharmazeutisch akzeptablen Vehikel enthalten.

**Patentansprüche für folgenden Vertragsstaat : GR**

1. Neue tricyclische Verbindungen der allgemeinen Formel

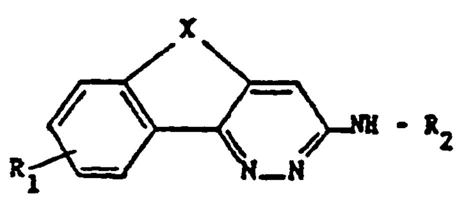

(I)

in welcher

— X ein Sauerstoff- oder Schwefelatom darstellt, oder aber X für eine Gruppe —$OCH_2$— oder eine Gruppe —$SCH_2$— steht,

— $R_1$ Wasserstoff oder ein Halogenatom, vorzugsweise Chlor, darstellt,

— $R_2$ darstellt :

* eine Gruppe

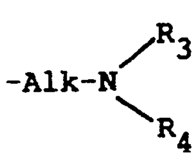

,

worin :

- Alk für eine gerade oder verzweigte Alkylengruppe mit 2 bis 5 Kohlenstoffatomen steht,
- $R_3$ und $R_4$ jeweils unabhängig Wasserstoff, eine Niedrigalkylgruppe mit 1 bis 4 Kohlenstoffatomen

darstellen, oder aber $R_3$ und $R_4$ mit dem Stickstoffatom, an das sie gebunden sind, eine cyclische Aminogruppe mit 5 oder 6 Ketten, die gegebenenfalls ein zweites Heteroatom umfaßt, bilden, oder

* eine Gruppe

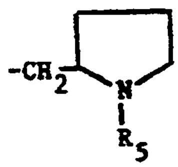
,

worin $R_5$ eine Niedrigalkylgruppe mit 1 bis 4 Kohlenstoffatomen darstellt ; sowie deren Salze mit pharmazeutisch akzeptablen Mineral- oder organischen Säuren.

2. Verbindungen nach Anspruch 1 der Formel (I), in welcher die Gruppe $R_2$

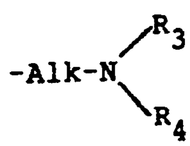 bezeichnet, worin 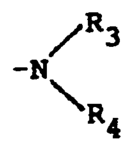

eine 1-Pyrolidinyl-, Piperidino-, Morpholino- oder 1-Piperazinylgruppe darstellt.

3. Verfahren zur Herstellung der Verbindungen der Formel (I) nach Anspruch 1, dadurch gekennzeichnet, daß

— ein Ketoheterocyclus zwischen 60 und 150°C mit Ethylglyoxylat oder Glyoxylsäure hitzebehandelt wird zur Bildung des Hydroxyesters oder der Hydroxysäure

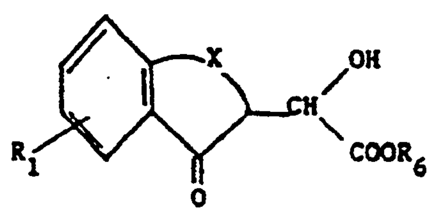
, worin $R_6$ H oder $C_2H_5$ ist,

(a)

— dieser Hydroxyester oder diese Hydroxysäure mit Hydrazinhydrat erhitzt wird, u.zw. entweder mit einem großen Überschuß des Hydrazinhydrats oder in einem Lösungsmittel, das für die Bildung des Pyridazons

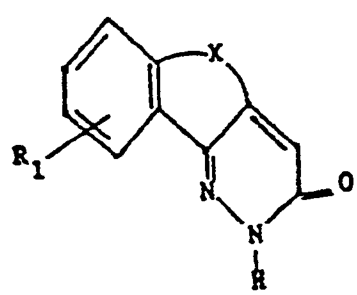

(b)

geeignet ist,

— das Pyridazon unter Rückfluß mit einem Überschuß an Phosphoroxychlorid oder -oxybromid behandelt wird, um das entsprechende Halogenderivat

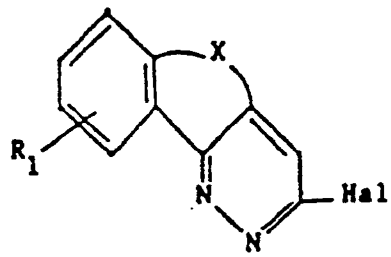

Hal = Cl, Br
(c)

zu bilden,

— das Halogenderivat durch Erhitzen mit einem Derivat der Formel $H_2N\text{-}R_2$ entweder in Gegenwart eines Überschusses des Derivats der Formel $H_2N\text{-}R_2$ oder in einem geeigneten Lösungsmittel, gegebenenfalls in Gegenwart eines Reaktionsaktivators zur Bildung der korrespondierenden Verbindung (I) substituiert wird ;

— gegebenenfalls die Verbindung (I) nach einem bekannten Verfahren in das Salz übergeführt wird.

4. Verfahren nach Anspruch 3, dadurch gekennzeichnet, daß das Hydrazinhydrat mit einem Hydroxyester oder einer Hydroxysäure (a) in einem Hydroxyllösungsmittel, wie n-Butanol oder Ethanol, erhitzt wird.

5. Verfahren nach einem der Ansprüche 3 oder 4, dadurch gekennzeichnet, daß die Reaktion des Halogenderivats (c) mit dem Derivat $H_2N\text{-}R_2$ in einem Hydroxyllösungsmittel, wie n-Butanol, oder in Dimethylformamid erfolgt.

6. Verfahren nach einem der Ansprüche 3 bis 5, dadurch gekennzeichnet, daß die Reaktion des Halogenderivats (c) mit dem Derivat $H_2N\text{-}R_2$ in Gegenwart von Ammoniumchlorid erfolgt.

7. Verfahren zur Herstellung von pharmazeutischen Zusammensetzungen, dadurch gekennzeichnet, daß es darin besteht, daß eine wirksame Dosis von mindestens einer Verbindung der Formel (I), wie in Anspruch 1 definiert, mit einem pharmazeutisch akzeptablen Vehikel vermischt wird.

**Patentansprüche für folgende Verstragsstaat : ES**

1. Verfahren zur Herstellung von neuen tricyclischen Verbindungen der allgemeinen Formel

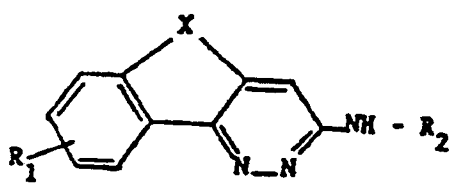

(I)

in welcher

— X ein Sauerstoff- oder Schwefelatom darstellt, oder aber X für eine Gruppe —$OCH_2$— oder eine Gruppe —$SCH_2$— steht,

— $R_1$ Wasserstoff oder ein Halogenatom, vorzugsweise Chlor, darstellt,

— $R_2$ darstellt :

* eine Gruppe

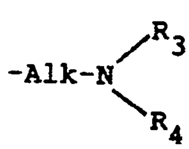

,

worin :

• Alk für eine gerade oder verzweigte Alkylengruppe mit 2 bis 5 Kohlenstoffatomen steht,

• $R_3$ und $R_4$ jeweils unabhängig Wasserstoff, eine Niedrigalkylgruppe mit 1 bis 4 Kohlenstoffatomen darstellen, oder aber $R_3$ und $R_4$ mit dem Stickstoffatom, an das sie gebunden sind, eine cyclische Aminogruppe mit 5 oder 6 Ketten, die gegebenenfalls ein zweites Heteroatom umfaßt, bilden, oder

* eine Gruppe

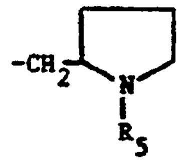 ,

worin $R_5$ eine Niedrigalkylgruppe mit 1 bis 4 Kohlenstoffatomen darstellt ; sowie deren Salzen mit pharmazeutisch akzeptablen Mineral- oder organischen Säuren, dadurch gekennzeichnet, daß es darin besteht, daß

— ein Ketoheterocyclus zwischen 60 und 150°C mit Ethylglyoxylat oder Glyoxylsäure hitzebehandelt wird zur Bildung des Hydroxyesters oder der Hydroxysäure

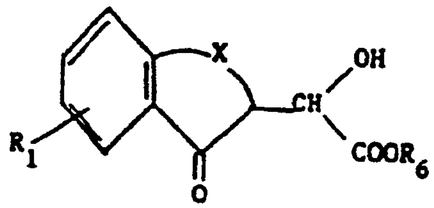 , worin $R_6$ H oder $C_2H_5$ ist,

(a)

— dieser Hydroxyester oder diese Hydroxysäure mit Hydrazinhydrat erhitzt wird, u.zw. entweder mit einem großen Überschuß des Hydrazinhydrats oder in einem Lösungsmittel, das für die Bildung des Pyridazons

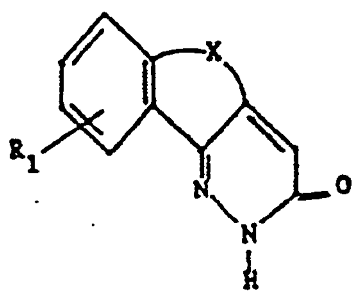

(b)

geeignet ist,

— das Pyridazon unter Rückfluß mit einem Überschuß an Phosphoroxychlorid oder -oxybromid behandelt wird, um das entsprechende Halogenderivat

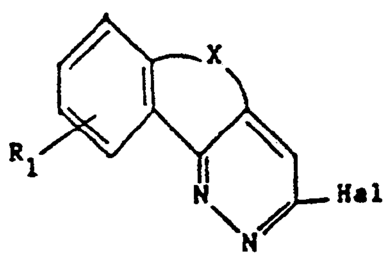

Hal = Cl, Br

(c)

zu bilden,

— das Halogenderivat durch Erhitzen mit einem Derivat der Formel $H_2N$-$R_2$ entweder in Gegenwart eines Überschusses des Derivats der Formel $H_2N$-$R_2$ oder in einem geeigneten Lösungsmittel, gegebenenfalls in Gegenwart eines Reaktionsaktivators zur Bildung der korrespondierenden Verbindung (I) substituiert wird ;

— gegebenenfalls die Verbindung (I) nach einem bekannten Verfahren in das Salz übergeführt wird.

2. Verfahren nach Anspruch 1, dadurch gekennzeichnet, daß das Halogenderivat (c) durch Erhitzen mit einem Derivat der Formel $H_2N$-$R_2$ substituiert wird, in welcher $R_2$ die Gruppe

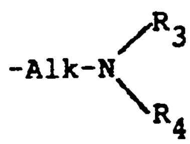 bezeichnet, worin 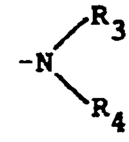

eine 1-Pyrrolidinyl-, Piperidino-, Morpholino- oder 1-Piperazinylgruppe darstellt.

3. Verfahren nach einem der Ansprüche 1 oder 2, dadurch gekennzeichnet, daß das Hydrazinhydrat mit einem Hydroxyester oder einer Hydroxysäure (a) in einem Hydroxyllösungsmittel, wie n-Butanol oder Ethanol, erhitzt wird.

4. Verfahren nach einem der Ansprüche 1 bis 3, dadurch gekennzeichnet, daß die Reaktion des Halogenderivats (c) mit dem Derivat $H_2N$-$R_2$ in einem Hydroxyllösungsmittel, wie n-Butanol, oder in Dimethylformamid erfolgt.

5. Verfahren nach einem der Ansprüche 1 bis 4, dadurch gekennzeichnet, daß die Reaktion des Halogenderivats (c) mit dem Derivat $H_2N$-$R_2$ in Gegenwart von Ammoniumchlorid erfolgt.

6. Verfahren zur Herstellung von pharmazeutischen Zusammensetzungen, dadurch gekennzeichnet, daß es darin besteht, daß eine wirksame Dosis von mindestens einer Verbindung der Formel (I), hergestellt nach einem der Ansprüche 1 bis 5, mit einem pharmazeutisch akzeptablen Vehikel vermischt wird.